(19) **Europäisches Patentamt**
**European Patent Office**
**Office européen des brevets**

(11) **EP 3 352 135 B1**

(12) # EUROPEAN PATENT SPECIFICATION

(45) Date of publication and mention
of the grant of the patent:
**11.09.2019 Bulletin 2019/37**

(51) Int Cl.:
**G06T 7/11** *(2017.01)* **G06T 7/136** *(2017.01)*

(21) Application number: **18151415.9**

(22) Date of filing: **12.01.2018**

(54) **METHOD AND APPARATUS FOR SEGMENTATION OF BLOOD VESSELS**

VERFAHREN UND VORRICHTUNG ZUR SEGMENTIERUNG VON BLUTGEFÄSSEN

PROCÉDÉ ET APPAREIL DE SEGMENTATION DES VAISSEAUX SANGUINS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.01.2017 PT 109864**

(43) Date of publication of application:
**25.07.2018 Bulletin 2018/30**

(73) Proprietor: **INESC TEC - Instituto de Engenharia
de Sistemas e
Computadores, Tecnologia e Ciência
4200-465 Porto (PT)**

(72) Inventors:
• **TERROSO DE ARAÚJO, Ricardo Jorge**
**4200-465 Porto (PT)**
• **PINTO DE OLIVEIRA, Hélder Filipe**
**4200-465 Porto (PT)**

(74) Representative: **Harrison, Robert John
24IP Law Group
Sonnenberg Fortmann
Patent- und Rechtsanwälte
Postfach 33 08 65
80068 München (DE)**

(56) References cited:
• **LANGE CHRISTOPHER J ET AL: "Automating
Perforator Flap MRA and CTA Reporting",
JOURNAL OF DIGITAL IMAGING,
SPRINGER-VERLAG, CHAM, vol. 30, no. 3, 17
January 2017 (2017-01-17), pages 350-357,
XP036230459, ISSN: 0897-1889, DOI:
10.1007/S10278-017-9943-Z [retrieved on
2017-01-17]**
• **NIUMSAWATT VACHARA ET AL: "The
Pfannenstiel scar and its implications in DIEP flap
harvest: a clinical anatomic study", EUROPEAN
JOURNAL OF PLASTIC SURGERY,
HEIDELBERG, DE, vol. 39, no. 1, 5 January 2016
(2016-01-05), pages 41-48, XP035869473, ISSN:
0930-343X, DOI: 10.1007/S00238-015-1176-0
[retrieved on 2016-01-05]**

**Description**

Background to the Invention

Breast cancer is a malignant tumour with its origin in the breast tissue, as defined by the American Cancer Society. It is estimated that more than 230,000 new cases of breast cancer will affect women in the United States during 2016. This represents about 29% of all new cancer cases and 15% of all cancer deaths among women [1]. However, incidence rates vary around the world. In general, developed countries present higher rates of breast cancer than non-developed countries. In the latter, non-developed countries, breast cancer is the most common cause of cancer mortality while in the former, developed countries it is the second most common cause of cancer mortality, being exceeded by lung cancer. The developed countries possess more efficient early diagnosis and treatments which leads to a lower mortality rate (25%) than the verified mortality rate in non-developed countries (37%).

Women who were diagnosed with breast cancer have higher chance of suffering from anxiety and depression resulting from the fear of recurrence, body image disruption, sexual dysfunction and mortality concerns [2]. Breast conservative methods have shown to have equivalent survival rates to mastectomy [3]. However, mastectomy - removal of the breast - is still a highly recurrent procedure and has even been increasing in some institutions [4-6]. This might suggest that some patients consider the removal of the entire breast to be a safer approach to eliminate completely the tumour. The option to reconstruct the breast after the mastectomy makes this idea more viable. Reconstruction methods allow a surgeon to recreate the breast shape, improving the way how women feel about themselves and their image after their breast(s) was(were) removed.

Breast reconstruction rates vary greatly according to the country, region and socioeconomic background of the patient [7]. The so-called deep inferior epigastric perforator (DIEP) flap has become a state-of-art technique for autologous tissue breast reconstruction [8]. The DIEP flap is a type of breast reconstruction in which blood vessels, called deep DIEP, as well as the skin and fat connected to them, are removed from the lower abdomen and transferred to the chest to reconstruct the breast after mastectomy without the sacrifice of any of the abdominal muscles.

Medical imaging has been playing a role in the breast reconstruction techniques since microsurgery started to be required to perform techniques such as the DIEP flap. The viability of these DIEP flaps is related to several features of the perforator(s) included in the DIEP flaps [9]. Preoperative imaging allows the clinician to plan the surgery according to the findings extracted by the clinician.

No algorithm is known from the literature focusing on the objective extraction of those relevant characteristics or even the segmentation of the deep inferior epigastric artery (DIEA) perforators.

Examples of the vessel segmentation include International Patent Application No. WO 2014/162263 (Philips) which uses a sequence of time series angiographic 2D images of a vascular structure that are obtained after injection of a contrast agent. A data processing unit is configured to determine an arrival time index of a predetermined characteristic related to the injection of the contrast agent, for ach of a plurality of determined pixels along the time series, and to compute a so-called connectivity index for each of the plurality of the determined pixels based on the arrival time index. The data processing unit generates segmentation data of the vascular structure from the plurality of the determined pixels. The segmentation data is based on the connectivity index of the pixels.

US Patent 8,768,436 (Hitachi Medical) teaches a method of processing X-ray CT images of a coronary artery region and a cardiac muscle region. This enables the effect of infarction or constrictions in the cardiac muscle region to be visually recognised.

Neither of these patent documents teaches a method that is suitable for vessel segmentation of DIEPs, in particular for the use in breast reconstruction techniques.

Vessel segmentation algorithms usually follow common principles and assumptions that stand true for different types of the vessels. A thorough description of the main approaches regarding three-dimensional (3D) vessel segmentation was made by Lesage et al. [10].

One approach to vessel segmentation is disclosed in "Automating Perforator Flap MRA and CTA Reporting", by Lange et al, J. Digit. Imaging (2017), 30:350-357 which teaches a method of segmentation in which an operator marks the vessels manually on an image.

Summary of the Invention

This document discloses a method for segmentation of blood vessels using a plurality of images with a plurality of voxels. The method comprises acquiring a plurality of images representing axial slices through a region of interest, for example the abdomen, and defining a fascia layer between a muscular region and a subcutaneous region by defining a boundary between high intensity regions and low intensity regions in the plurality of images. The method further comprises determining a first landmark of the blood vessel, followed by calculating a subcutaneous path between the landmark of the blood vessel and the fascia layer; and then calculating an intramuscular path between the fascia layer and a second landmark. The first landmark is on one side of the fascia layer and the second landmark is on the other side of the fascia layer.

In order to segment the blood vessel effectively greyscales in the acquired plurality of images are reduced to binary images and/or furthermore artefacts on the images, such as the skin or vacancies in an otherwise continuous feature are removed.

The subcutaneous path is calculated using a tracking procedure, which comprises analysis of the blood vessel direction along the blood vessel. The centre of the vessel is determined from changes in intensity of voxels in the plurality of acquired images. The intramuscular path is calculated using a minimum cost path method by analysis of the voxels and determining a least cost route given by the vesselness of the voxels.

An apparatus for the segmentation of a blood vessel is also disclosed. The apparatus includes a database for storing a plurality of images of axial slices of a region of interest and a processor for analysing the plurality of images with a software carrying out the method.

Description of the Figures

Fig. 1a) and 1c) shows CTA axial slices (not adjacent) with the region of interest inside the white boxes; and Figs. 1b) and 1d) shown the corresponding regions of interest where important structures or areas are labelled.

Fig. 2 shows an outline of the method of the current invention.

Fig. 3 shows a representation of the anterior abdominal anatomy in the sagittal plane.

Fig. 4 shows original images (in the left column) and corresponding segmentations obtained using the threshold given by Otsu's method (in the right column) [11].

Fig. 5 shows an outline of the image correction methods.

Fig. 6 shows output of the modules Filling Operation (left column) and Raw fascia segmentation (right column) for the example images used in Figure 4.

Fig. 7 shows preliminary fascia segmentations in sagittal slices (left column) and corresponding final segmentations (right column).

Fig. 8 shows a diagram representing the prediction obtained by analysing the local gradients and the correction measure extraction step.

Fig. 9 shows a template for locating the ridge point.

Fig. 10 shows a centreline point correction measure, a) initial cross section image with gradient vector field superimposed; b) inner product responses; c) centre estimation.

Fig. 11 shows different slices of a patient volume (left column) and corresponding costs obtained by applying the transform to the Frangi vessel probabilities (right column) [16]. The arrows locate intramuscular vessels.

Fig. 12 shows a representation of the process to obtain the line which goes along the axial cross-section of the vessel. $v_{\|}A$ is the projection of v into the plane A.

Fig. 13 shows an outline of the apparatus used.

Detailed description of the Invention

This document describes a method and apparatus used to extract the relevant characteristics of perforators (blood vessels that penetrate an organ) as well as validation of a local gradient based tracking procedure to detect the subcutaneous region of perforators; validation of an A* based search using as costs the transformed Frangi Vesselness [16] to extract the intramuscular course of the perforators.

The Champalimaud Foundation in Lisbon provided computer tomography angiography (CTA) volumes from twenty different patients in digital imaging and communications in medicine (DICOM) format and comprise a number of axial slices, for example fifty or more depending on the resolution required, which are imaged perpendicular to the long axis of a body of a patient. The CTA is a technique known in the art used to visualise arteries and veins in a body of a patient.

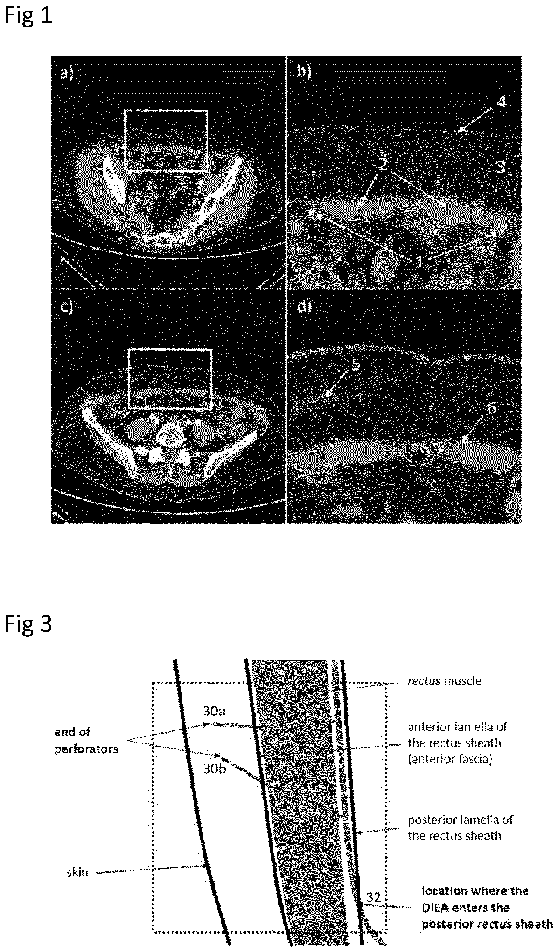

The CTA volumes provide information from the entire abdominal region of the patients. This document teaches a method that focuses on the anterior abdominal wall region, since it is there that DIEP perforators arise. Figure 1 shows some examples of the region of interest along with labels of the existing structures. Figs. 1a and 1c show CTA axial slices (not adjacent) with the region of interest inside the white boxes. Figs. 1b and 1d show the corresponding regions of interest in which important structures or areas are labelled: 1 - right and left DIEA, 2 - rectus abdominis muscle, 3 - subcutaneous region, 4 - skin tissue, 5 - subcutaneous portion of a perforator, 6 - intramuscular portion of a perforator.

In terms of height, the volume of interest starts at the region at which the DIEA's enter the posterior lamella of the rectus abdominis muscle sheath (see Fig. 1b) and ends a little above the umbilicus area. It is not expected to find DIEA perforators above this section.

The course of the perforators (both subcutaneous and intramuscular) was provided by an expert as "Ground truth" landmarks - i.e. directly observed information. The Champalimaud Foundation provided a medical report for each of the patients. A description of the existing perforators was made, for example, by a radiologist. The description included the calibre (inside diameter) of the perforators, sites where the perforators leave the fascia, subcutaneous course orientation and intramuscular course tortuosity and length.

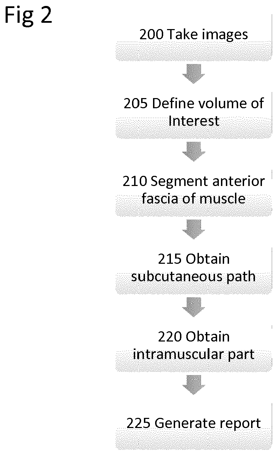

Fig. 2 shows the method according to one aspect of this invention. The first step 200 involves acquisition of images and then requires that the radiologist (for example) in step 205 defines the volume of interest, by manually selecting the sites where the DIEA's enter the posterior lamella of the rectus abdominis muscle sheath and the endpoint of each of the perforator (see Fig. 3). These represent the landmarks which are used by the method of this disclosure from which to calculate the path of the vessel. It will be seen that there are two endpoints 30a and 30b of the perforators shown in Fig. 3, which form the first landmarks, and the position 32 where the DIEA enters the posterior lamella of the rectus abdominis muscle sheath forms the second landmark. Fig. 3 represents a simplification as there will be 6-8 perforators in a woman.

This is followed with a method that automatically extracts the perforator vessels, with the aid of those landmarks. It can be seen in Fig 1d) that the subcutaneous and intramuscular regions of the perforator present very distinct SNRs. This suggests a fast track approach to obtain the subcutaneous path in step 215 and a more complex minimum cost path method to extract the intramuscular path in step 220.

To know how to separate both regions we first segment the anterior fascia of the muscle in step 210. Finally, after the extraction of the existing perforators, we generate in step 225 a report containing the relevant characteristics of each of the perforators.

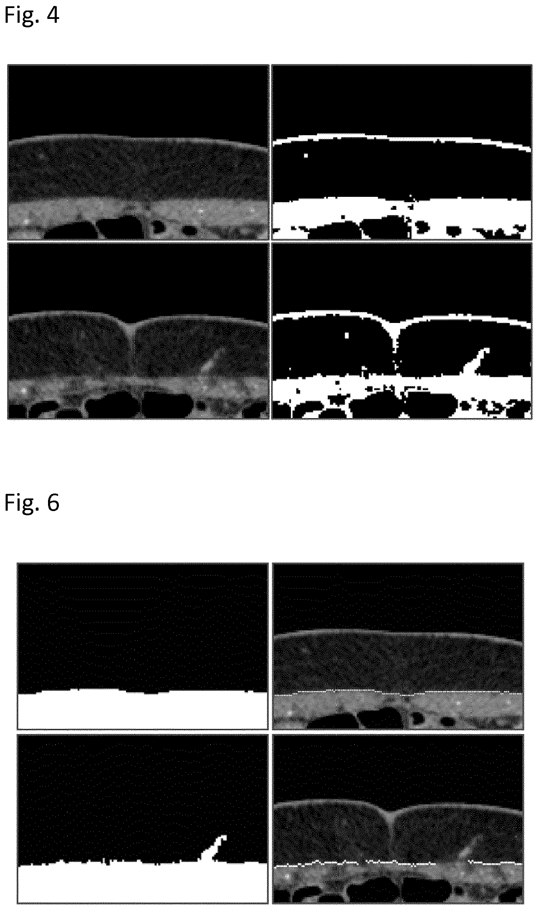

The anterior fascia is a thin layer of tissue that separates the rectus abdominis muscle from the underlying soft tissues in the subcutaneous region. In terms of image intensities of the CTAs, it is believed that the anterior fascia cannot be easily distinguished from the rectus abdominis muscle. The anterior fascia, as noted above, is considered to be the boundary between this rectus abdominis muscle and the subcutaneous region and is characterized by a transition from pixels in the images with a low intensity (indicating the subcutaneous region) to pixels with higher intensity (indicating the rectus abdominis muscle, i.e. muscular region), which exists over all the columns of each axial slice, when considering only the region of interest. Thus, Otsu's method [11] for reduction of greyscale images to binary images was applied to the region of interest of each of the axial slices generated from the CTA, with the goal of distinguishing the muscle from the subcutaneous region (see Figure 4 for example results). The original greyscale images are shown in the left column of Fig. 4 and corresponding segmentations (binary images) obtained using the threshold are given by Otsu's method [11] and shown in the right column of Fig. 4.

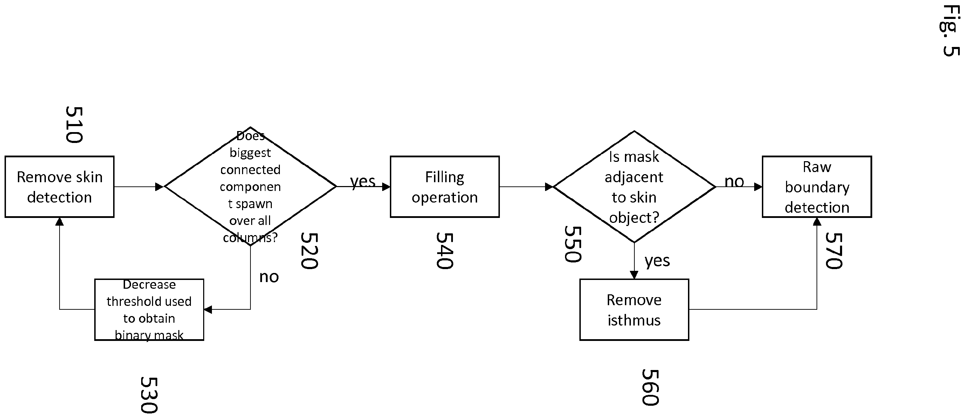

To obtain a preliminary segmentation of the fascia, the method shown in Figure 5 is followed. In step 510, any skin detection on the image is removed. The skin is an artefact on the image and can be seen as a thin line on the images shown in Fig. 4. The effect of the removal of the skin can be seen on the left-hand images of Fig. 6. The skin is the region in the images that separates the soft tissues from the outside of the patient and is therefore not of interest. The test in step 520 determines whether a single component extends over all of the columns. Should that not be the case, then the threshold level for the binary images is changed in step 530, and any detected skin removed in step 510, before repeating step 520.

A step 540 involves a filling operation to fill in any gaps in the images due missing (white pixels) in components that are clearly present in the image. This can be seen in the left hand side of Fig. 6 in which the filling operation has been completed.

Suppose now that another artefact or object is detected in step 550 between the component and the skin, as can be seen in the bottom half of Fig. 4. This object is termed an isthmus and needs to be removed from the image in step 560. This can be done by removing pixels detected by calculating the horizontal derivatives of the image intensity. Finally, step 570 is responsible for keeping contours connected. A connected contour is considered to a line in which every dark pixel in the 8-neighborhood or Moore neighbourhood is connected, i.e. every other pixel connected to edge or corner of one pixel). Suppose that some contours are found after processing the images, which are clearly not part of the boundary separating the muscle from the soft tissue in the subcutaneous region - a sort of "orphan curve" and another unwanted artefact. Then these artefacts or curves need to be deleted from the image. An empirically-based decision was made that as long as the orphan curve connected was less than $n$ pixels, where $n$ was empirically set to 11, then the missing curve could be deleted.

To achieve the final segmentation, which is a complete and smoother version of the preliminary one, the new fascia estimations are set as the output of local regressions using the preliminary detection neighbours on the sagittal plane. It is known that in sagittal slices, the boundary between the muscle and the subcutaneous region is usually very smooth. For each row of each sagittal slice of our volume of interest, a new fascia point ($p_{row}$, $p_{col}$) is given by the equation:

$$p_{col} = P(p_{row}) \qquad (1)$$

where $P$ is a local regression model based on a bisquare objective function which has into account the sagittal neighbours contained in the range $[p_{row} - n, p_{row} + n]$, being $n$ expressed by:

$$n = k\frac{m}{s} \tag{2}$$

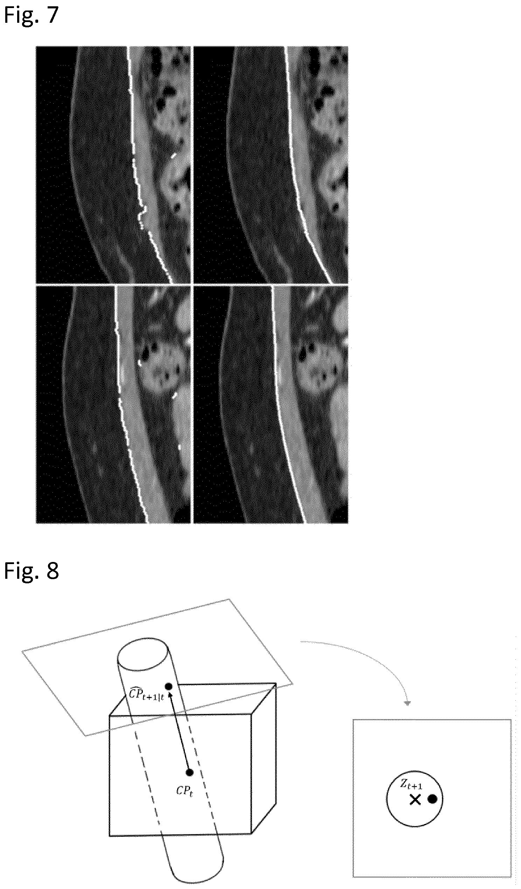

$s$ is the distance in millimetres between consecutive pixels, characteristic of the volume (same in every direction of the volume after interpolation of data), $m$ is the size of the biggest structures to be neglected, also in millimetres. This is done to smooth the data in the images in order to remove the influence of the biggest structure from affecting the fascia segmentation. In the data sets of this example, the vessel calibre is the biggest structure that should be neglected. $k$ is a constant. This last constant $k$ is the amount of data which has to be considered to remove the influence of the structure, e.g. the vessel calibre, from the image. In this work, $m = 5$ was considered, and $k = 5$ was empirically obtained. In other words, to remove or neglect a structure that appears in three of the pixels in the image, it is necessary to use data from 3 time $k$ pixels for this calculation, i.e. from 15 neighbouring pixels. The results of this approach can be visualized in Figure 7. It can be seen that the result of this equation is to produce the smooth contour.

The detection of the vessel will now be explained.

The subcutaneous path (step 210) can be calculated given that the end point of each perforator (the first landmarks) and the fascia layer are known. A tracking procedure is used to estimate new centreline points along the vessel until the fascia layer is reached. The centreline points are calculated according to the local vessel direction:

$$CP_{t+1} = CP_t + s\hat{v} \tag{3}$$

$CP_t$ is the centreline point estimated at iteration $t$, $s$ is a scalar which controls the step given between consecutive centreline points ($s = 1$ was used) and $\hat{v}$ is the unit vector pointing towards the local vessel direction. The latter unit vector $\hat{v}$ is estimated through the analysis of the local gradient vectors, based on Agam et al. [12]. The vessel direction $\hat{v}$ is the one which minimizes the squared projection of the local gradient vectors into v:

$$E(\boldsymbol{v}) = \frac{1}{n}\sum_{i=1}^{n}((\boldsymbol{g}_i)^T\boldsymbol{v})^2 = \boldsymbol{v}^T\left(\frac{1}{n}\sum_{i=1}^{n}(\boldsymbol{g}_i)(\boldsymbol{g}_i)^T\right)\boldsymbol{v} \tag{4}$$

where $n$ is the number of local gradient vectors and $g_i$ is the ith gradient vector. The letter $i$ indicates the index of the local gradient vector and goes from 1 to $n$. It will be realised that the number of the local gradient vectors depends on the size of the window used. For example, if the window is of size $3 \times 3 \times 3$, then there will be 27 local gradient vectors characterising the neighbourhood of the voxel.

By denoting

$$G \equiv \left(1/\sqrt{n}\right)[\boldsymbol{g}_i, \dots, \boldsymbol{g}_n] \tag{5}$$

the previous expression becomes $E(v) = v^T G G^T v$, where $GG^T$ is a 3x3 correlation matrix. As shown by Agam et al. [12], the minimum of $E(v)$ is obtained by the eigenvector of $GG^T$ belonging to its smallest eigenvalue.

Then, to estimate the value of $CP_{t+1}$, the local gradient vectors contained in the neighbourhood of $CP_t$ are used. This neighbourhood is given by a $p \times p \times p$ window, where $p = 7$ was empirically selected for this work. In this example, the number of local gradient vectors is therefore $7 \times 7 \times 7 = 343$.

This method allows the finding of the local direction of a vessel which goes through the $p \times p \times p$ window. It will be observed that this method does not guarantee that the estimated centreline point is near the centre of the vessel. To correct this issue, an additional measure was used, which was incorporated in the framework through a Kalman filter [13]. It relies on the assumption that voxels on the centre of the vessel have higher intensity and that the intensity of the voxels decrease as the distance to the centre increases. In a 2D image of the cross section of the vessel, it is then expected that the centre location, $Z$, can be found by analysing the divergence of the gradient vector field.

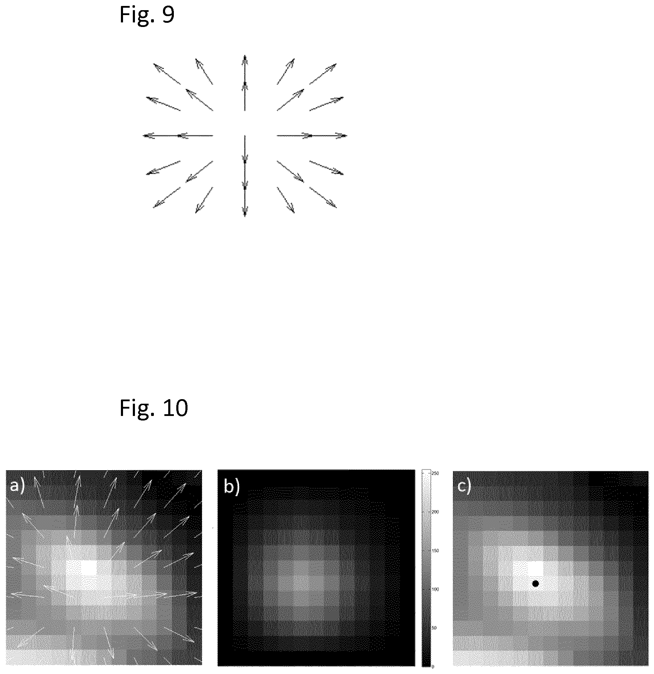

After predicting the position of the new centreline point $\widehat{CP}_{t+1|t}$ using the local gradient vectors information, the plane which contains that centreline point and is orthogonal to the vessel direction $\overset{\wedge}{v}$ is obtained (see Figure 8). It is expected that this plane includes a roughly circular brighter region which is the 2D cross section of the vessel. The gradient vector field is calculated [14] and its similarity to the template represented in Figure 9 is assessed through cross-correlation:

$$(f * g)[n] \overset{\text{def}}{=} \sum f^{*}[m]\, g[n + m] \tag{6}$$

where $f$ and $g$ represent the gradient orientation vector field and template vector field, respectively, and $f^{*}$ is the complex conjugate of $f$. The centre location estimation $Z_{t+1}$ corresponds to the maximum response location (see Figure 10). The estimated centreline point is the output of a Kalman filter fusing $\widehat{CP}_{t+1|t}$ and a correction measure $Z_{t+1}$ whenever the correction measure is available. In this work, we took that correction measure every 5 iterations. It would at least be possible to calculate the correction measure more frequently, but this would increase the computational cost.

The calculation of the intramuscular path will now be explained. As already stated, the images representing the intramuscular course of the perforators commonly have a very low SNR. Thus, general tracking procedures are not adequate for this task of determining the intramuscular course. The use of a minimum cost path method is proposed to find the intramuscular vessel pathway between the site where the perforator reaches the fascia and a manually identified DIEA second landmark. As explained above, this is where the perforator penetrates the posterior lamella of the rectus muscle sheath. Thus, the problem becomes constrained to finding a path that connects two voxels, leading to a decrease in the computational effort required. Even then, using a plain Dijkstra search method for such task might lead to visiting a high number of voxels. The inventors propose the use of the A* algorithm [15], as the A* algorithm includes a heuristic to improve the search performance. At each iteration, the A* search algorithm expands the path which minimizes the following expression:

$$f(n) = g(n) + h(n) \tag{7}$$

where $n$ is the last node on the path, $g(n)$ is the cost of the path from the start node to $n$, and $h(n)$ is the heuristic that estimates the cost of the cheapest path from $n$ to the goal. In this work, the Euclidean distance between n and the target voxel is used as the heuristic function.

To find the desired pathways, lower costs must be given to vessel voxels. The cost of travelling from one node to another is given by the following expression:

$$c_{n,n+1} = d_{n,n+1} \cdot C(n + 1) \tag{8}$$

where $n$ is the current node, n + 1 is the neighbour node, $d_{n,n+1}$ is the Euclidean distance between those nodes, and $C(n + 1)$ is the terrain cost of the neighbour node. The costs volume is given by:

$$C(n) = \begin{cases} 2 - F(n) & if\ F(n) > 0 \\ 10 & if\ F(n) = 0 \end{cases} \tag{9}$$

where $F(n)$ is the Frangi vesselness [16] at voxel $n$ normalized to the range [0,1]. This formulation gives relatively high costs to voxels which do not belong to vessels ($F(n) = 0$), and costs in the range [1, 2] otherwise, in order to guarantee that the heuristic is admissible.

The Frangi method [16] for the calculation of vesselness aims at analysing the local intensity structure along the image and enhancing tubular-shaped objects in the image.

It is known that a Hessian matrix is a square matrix of second-order partial derivatives of a function. In the context of the Frangi method [16], the function is the intensity distribution along a 2D image or a 3D volume. Considering a 3D volume, for each voxel we calculate the Hessian matrix, H, as follows:

$$H = \begin{bmatrix} V * \dfrac{\partial^2 G}{\partial^2 x} & V * \dfrac{\partial^2 G}{\partial x \partial y} & V * \dfrac{\partial^2 G}{\partial x \partial z} \\[2ex] V * \dfrac{\partial^2 G}{\partial x \partial y} & V * \dfrac{\partial^2 G}{\partial^2 y} & V * \dfrac{\partial^2 G}{\partial y \partial z} \\[2ex] V * \dfrac{\partial^2 G}{\partial x \partial z} & V * \dfrac{\partial^2 G}{\partial y \partial z} & V * \dfrac{\partial^2 G}{\partial^2 z} \end{bmatrix} \qquad (10)$$

where * denotes the convolution operation, $V$ is the local volume, $G$ is the Gaussian, and its sigma determines the scale of the local structure being analysed. Frangi's method is multiscale since, for each voxel, it calculates $H$ at different scales.

For each of these matrices calculated for each of the voxels, an eigenvalue analysis is performed, obtaining three eigenvectors and their corresponding eigenvalues. The eigenvectors associated to the two eigenvalues of highest absolute value point towards the directions of higher local intensity curvature (note however that these eigenvectors point in the directions that are normal to the vessel direction) and the last eigenvector points in the direction that is normal to those two (along vessel direction).

Then, for each voxel of our volume, one will have $N$ pairs of eigenvalues, where $N$ is the number of scales we have used. Each pair has three eigenvalues - $\lambda_1, \lambda_2, \lambda_3$ - of increasing absolute values, respectively. The Frangi vesselness measure [16] is calculated for each of those pairs:

$$F(n) = \begin{cases} 0 & \text{if } \lambda_2 > 0 \text{ or } \lambda_3 > 0 \qquad (8) \\[1ex] \left[ 1 - \exp\left( -\dfrac{R_A{}^2}{2\alpha^2} \right) \right] \left[ \exp\left( -\dfrac{R_B{}^2}{2\beta^2} \right) \right] \left[ 1 - \exp\left( -\dfrac{S^2}{2c^2} \right) \right] \end{cases}$$

$R_A$, $R_B$, $S$ are obtained through relations between the eigenvalues, as discussed in Frangi [16] The underlying idea is to produce higher probabilities of the presence of the vessel at tubular shaped regions and lower probabilities at blob-like and constant regions. The other constants are empirically derived from Frangi method and are present in the formula to control the sensitivity of each parameter. The response at the different scales is combined by, for each pixel, selecting the higher value of the Frangi vesselness measures. Then, this method is able to enhance vessels at varying widths due to the use of different scales. It will be understood that lower scales enable detection of the vessels with narrower widths.

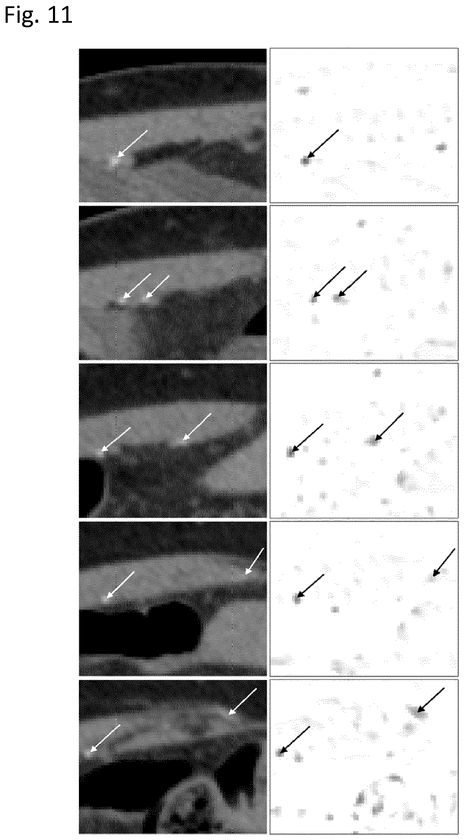

Figure 11 shows slices that belong to one of the volumes from the database, and the corresponding costs obtained. The arrows point to the intramuscular vessels that should be enhanced in order to be possible to correctly extract the vessel pathways of interest. We conclude that vessels are being differentiated, as well as some noisy areas. Even then, the cumulative path cost term will favour paths going through vessels as they generally contain consecutive low cost voxels.

The method enables, in an objective and reproducible manner, determination of the relevant characteristics of each perforator for surgery planning. Hence, after extracting the vessels, we still need to reproduce a medical report describing the following points.

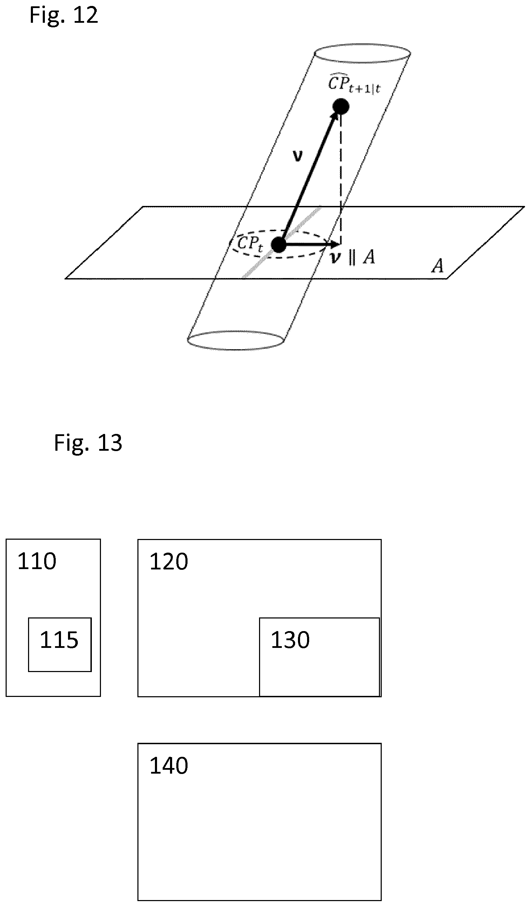

At each tracked point of the subcutaneous region, we extract a line which passes through the tracked point of the subcutaneous region and cuts along the cross section of the perforator at the axial plane (see Figure 12 for an illustration of the process). This line is used to obtain the intensity profile of the cross section in a denoised version of the original image [17]. To measure the calibre, a Gaussian is fitted to the profile in a least-squares fashion and the width in mm covered by the 85% confidence interval is taken. This value was empirically regarded as the best for the data contained in the database. The final perforator calibre is the mean of the measures taken at each tracked point.

Site where the perforator leaves the fascia. This is regarded as the location where our tracking procedure stops due to reaching the fascia.

Orientation of subcutaneous course. To know if the perforator is externally or medially oriented and ascending or descending we build histograms of the orientation vectors between consecutive centreline points and use a classifier previously trained with the available annotated data.

Length and tortuosity of intramuscular course. The length of an intramuscular pathway is given by the sum of the distance between consecutive points. To decide if the path is short or long, a threshold learnt with the available annotated data is used. Regarding the tortuosity of a vessel, the inventors calculate tortuosity metrics [18] and use a classifier that outputs one of two classes, tortuous or linear. Once again, the classifier was trained with the available annotated data.

Fig. 13 shows a non-limiting example of a system that can be used to carry out the method of this document. The system 100 comprises a database 110 having a plurality of images 115 of axial slices from the patient. The database 110 is connected to a processor 120 on which is running a software 130 to implement the method. A display device 140 is connected to the processor 120 and outputs the required results.

**Examples**

For each volume of the database from the Champalimaud Foundation, a manual annotation of the anterior fascia layer was made. For each axial slice of the volume, several pixels of the anterior fascia were selected and a linear interpolation used to obtain a complete fascia segmentation. This Ground Truth annotation was then compared to the segmentation that was obtained with the method outlined in this document. The mean Euclidean and Hausdorff distances were calculated and the results obtained are summarized in Table 1.

Table 1. Results obtained by the proposed fascia segmentation method. The mean Euclidean and Hausdorff distances between the segmentations and the manual annotations, and the average execution time, are shown.

| Euclidean distance (mm) | | Hausdorff distance (mm) | | Mean time (s) |
|---|---|---|---|---|
| GT $\rightarrow$ Seg | Seg $\rightarrow$ GT | GT $\rightarrow$ Seg | Seg $\rightarrow$ GT | |
| 0.49 $\pm$ 0.33 | 0.51 $\pm$ 0.40 | 1.52 $\pm$ 0.76 | 1.63 $\pm$ 1.15 | 636.21 $\pm$ 167.63 |

The voxel spacing differs between the volumes of the database, but in the majority of the cases it lies between 0.7 and 0.9 mm. This shows that the method presented in this disclosure was able to provide segmentations whose mean distance to the manual annotations was lower than the spacing between consecutive voxels. Using an Intel Core i7-4500U CPU 1.80@2.40GHz to run a MATLAB (R2014a) script, the average run time was 636 s for each volume.

Across the volumes of the database, 74 subcutaneous and 28 intramuscular perforator pathways were annotated by the expert. To initialize the vessel detection procedure of this document, the tracking was started at the Ground Truth landmark which was closer to the end of the perforator. After obtaining both regions paths, the Euclidean and Hausdorff distances from the Ground Truth annotations to the extracted paths were calculated, since the Euclidean distances are more sparse than the Hausdorff distances and calculating those metrics in the other way around would yield a larger error without true meaning. Table 2 summarizes the results obtained.

Table 2. Results obtained by using the proposed methodologies to detect the subcutaneous and intramuscular regions of the perforators.

| Region | Path error (mm) | | Mean time (s) |
|---|---|---|---|
| | Euclidean distance | Hausdorff distance | |
| subcutaneous | 1.35 $\pm$ 0.46 | 2.98 $\pm$ 1.46 | irrelevant |
| intramuscular | 1.06 $\pm$ 0.32 | 2.44 $\pm$ 0.92 | 15.00 $\pm$ 14.76 |

Considering that the spacing between voxels is around 0.7-0.9 *mm,* the method of this disclosure extracted paths with an average error larger than a pixel. It is believed that two reasons explain this error. First, the annotation provided by the radiologist is not in the form of a skeleton, while the results presented above are. This means that, even if the retrieved path is a perfect skeleton of the vessel, the provided Ground Truth annotations will lead to a significant error when performing the comparison. The second reason, related only to the subcutaneous path detection and explaining why the error was larger there, comes from the fact that the gradient based tracking algorithm was not adequate to follow correctly courses adjacent to the muscle area (vessel and muscle appear merged in terms of intensities). Although it does not occur very commonly, it also explains the increased error. It is also the reason why the mean Hausdorff distance reached a relatively high value, 2.98 *mm.*

The estimations of the calibre and the location where the perforator leaves the anterior fascia were also compared to the Ground Truth. Note that, in order to create the map of perforators, only the width and height of the location where the perforator leaves the fascia matters. Hence, the error was independently evaluated for both. Table 3 summarizes the results obtained.

The calibre estimation method reached a mean error which corresponds to less than half of the spacing between consecutive voxels. Note that the calibre Ground Truth available comes from reports which were produced by different medical personnel, increasing the subjectivity behind the process. Thus, more conclusive results could be produced if different experts annotated the same data, such that the inter-operator variability could be measured. Finally, in terms of stopping the tracking procedure at the correct location, one can notice that the error was higher in terms of width offset than height one. This happens because, the already explained behaviour where perforators occasionally move along the muscle, tends to occur through the axial plane. Then, stopping the tracking earlier due to the contamination of local gradient vectors by the presence of the muscle, commonly leads to a higher offset in width estimate than height.

Table 3. Results obtained by using the proposed methodologies to estimate the calibre and location where the perforator leaves the fascia.

| Calibre error | | Location error (mm) | |
|---|---|---|---|
| relative | Absolute (mm) | height | width |
| 15.4% | $0.35 \pm 0.27$ | $1.40 \pm 1.39$ | $1.72 \pm 1.49$ |

Using the method of this document, the characteristics of the DIEA perforators which are relevant for breast reconstructive surgery can be extracted. The method enables accurate segmentation of the fascia layer. This segmentation is used to divide detection of the perforators into two independent problems: detection of the subcutaneous courses and detection of the intramuscular courses. The subcutaneous courses were correctly extracted by a Kalman filter fusing the local gradient vectors information with a 2D cross section vessel centre estimation, in order to iteratively extract new centerline points. A mean error of 1.35 mm was achieved. The intramuscular courses were extracted by means of the Frangi vesselness based minimum cost path approach with a mean error of 1.06 *mm*.

Following the vessel detection step, it is possible to characterize each perforator according to the clinical relevant aspects. We achieved a mean subvoxel error when measuring the calibre and determined the site where the perforators leave the fascia with a mean error of approximately one and a half voxels. Besides, we proposed algorithms to obtain the orientation of the subcutaneous course of perforators and the length and tortuosity of their intramuscular region.

Considering the subcutaneous tracking procedure, attention should be given to the perforators which present a significant course along the fascia. This makes the tracking method unstable at that region due to the corrupted local gradient vectors and it commonly stops earlier than it should, not retrieving the real coordinates of the region where the perforator leaves the fascia.

In terms of calibre estimation, different radiologists should provide the respective Ground Truth, in order to measure the inter-operator variability and compare with the results achieved by the developed framework. Given the high subjectivity inherent to the calibre estimations, this is a very important point.

Finally, a software that renders the detected vessels and displays them with the volume data is developed.

**References**

1. Siegel, R., Miller, K., Jemal, A.: Global cancer statistics. A Cancer Journal for Clinicians. 65, 5{29 (2015)

2. Hewitt, M., Herdman, R., Holland, J.: Meeting psychosocial needs of women with breast cancer. The National Academies Press. (2004)

3. Lichter, A., Lippman, M., Danforth, D., Angelo, T., Steinberg, S., deMoss, E., Mac-Donald, H., Reichert, C., Merino, M., Swain, S., Cowan, K., Gerber, L., Bader, J., Findlay, P., Schain, W., Gorrell, C., Straus, K., Rosenberg, S., Glatstein, E.: Mastectomy versus breast-conserving therapy in the treatment of stage I and II carcinoma of the breast: a randomized trial at the National Cancer Institute. Journal of Clinical Oncology. 10, 976{983 (1992)

4. McGuire, K., Santillan, A., Kaur, P., Meade, T., Parbhoo, J., Mathias, M., Shamehdi, C., Davis, M., Ramos, D., Cox, C.: Are Mastectomies on the Rise? A 13-Year Trend Analysis of the Selection of Mastectomy Versus Breast Conservation Therapy in 5865 Patients. Annals of Surgical Oncology. 16, 2682{2690 (2009)

5. Dragun, A., Huang, B., Tucker, T., Spanos, W.: Increasing mastectomy rates among all age groups for early stage breast cancer: A 10-year study of surgical choice. Breast Journal. 18, 318{325 (2012)

6. Mahmood, U., Hanlon, A., Koshy, M., Buras, R., Chumsri, S., Tkaczuk, K., Cheston, S., Regine, W., Feigenberg, S.: Increasing national mastectomy rates for the treatment of early stage breast cancer. Annals of surgical oncology. 20, 1436{43(2013)

7. Wexelman, B., Schwartz, J., Lee, D., Estabrook, A., Ma, A.: Socioeconomic and Geographic Di_erences in Immediate Reconstruction after Mastectomy in the United States. The Breast Journal. 20, 339{346 (2014)

8. Cina, A., Salgarello, M., Barone-Adesi, L., Rinaldi, P., Bonomo, L.: Planning breast reconstruction with deep inferior epigastric artery perforating vessels: multidetector CT angiography versus Color Doppler US. Radiology. 255, 979{987 (2010)

9. Phillips, T., Stella, D., Rozen, W., Ashton, M., Taylor, G.: Abdominal wall CT angiography: a detailed account of a newly established preoperative imaging technique. Radiology. 249, 32{44 (2008)

10. Lesage, D., Angelini, E., Bloch, I., Funka-Lea, G.: A review of 3D vessel lumen segmentation techniques: Models, features and extraction schemes. Medical Image Analysis. 13, 819{845 (2009)

11. Otsu, N.: A threshold selection method from gray-level histograms. IEEE Transactions on Systems, Man, and Cybernetics. 9, 62{66 (1979)

12. Agam, G., Armato, S., Wu, C.: Vessel tree reconstruction in thoracic CT scans with application to nodule detection. IEEE Transactions on Medical Imaging. 24, 486{499 (2005)

13. Kalman, R.: A New Approach to Linear Filtering and Prediction Problems. Journal of Basic Engineering. 82, 35{45 (1960)

14. Oliveira, H., Cardoso, J., Magalhes, A., Cardoso, M.: A 3D low-cost solution for the aesthetic evaluation of breast cancer conservative treatment. Computer Methods in Biomechanics and Biomedical Engineering: Imaging & Visualization. 2, 90{106 (2014)

15. Hart, P., Nilsson, N., Raphael, B.: A formal basis for the heuristic determination of minimum cost paths. IEEE Transactions on Systems, Science, and Cybernetics. 4, 100 {107 (1968)

16. Frangi, A., Niessen, W., Vincken, K., Viergever, M.: Multiscale vessel enhancement Filtering. Medical Image Computing and Computer-Assisted Intervention. Lecture Notes in Computer Science. 1496, 130{137 (1998)

17. Kovesi, P.: Phase preserving denoising of images. DICTA 99: Fifth International/National Biennial Conference on Digital Image Computing, Techniques and Applications. 212{217 (1999)

18. Bullitt, E., Gerig, G., Pizer, S., Lin, W., Aylward, S.: Measuring tortuosity of the intracerebral vasculature from MRA images. IEEE Trans. Med. Imaging. 22, 1163{1171 (2003)

**Claims**

1. A method for segmentation of blood vessels comprising:

   acquiring (200) a plurality of images (115) with a plurality of voxels representing axial slices through a region of interest;
   defining (210) a fascia layer between a muscular region and a subcutaneous region by defining a boundary between high intensity images and low intensity images;
   determining a first landmark and a second landmark of a blood vessel, wherein the first landmark is on one side of the fascia layer and the second landmark is on the other side of the fascia layer;
   calculating (215) a subcutaneous path between the first landmark of the blood vessel and the fascia layer by an automatic tracking procedure comprising analysis of vessel direction along the blood vessel; and
   calculating (220) an intramuscular path between the fascia layer and the second landmark by analysis of the voxels and automatically determining a least cost route given by the vesselness of the voxels.

2. The method of claim 1, further comprising reducing greyscales in the acquired plurality of images to binary images.

3. The method of any of the above claims, further comprising removal (510, 560) of artefacts from the plurality of images.

4. The method of claim 3, wherein the artefacts in the image comprise pixels relating to skin, vacancies in an otherwise continuous feature.

5. The method of any of the above claims, wherein the analysis of the vessel direction is estimated by analysis of local gradient vectors.

6. The method of any of the above claims, wherein the centre of the blood vessel is determined from changes in intensity of the voxels in the plurality of acquired images.

7. The method of any of the above claims, wherein the vesselness of the voxels is determined according to the Frangi method.

8. An apparatus for the segmentation of a blood vessel comprising:

a database (110) for storing a plurality of images (115) of axial slices of a region of interest;
a processor (130) configured to analyse the plurality of images with a software carrying out the method according to one of claims 1 to 7; and
a display device (140) for outputting results from the software.

9. A computer program product stored on a non-tangible computer readable medium and comprising a plurality of instructions which, when executed by a processor (130), cause the processor (130) to perform the method of any one of the claims 1 to 7.

**Patentansprüche**

1. Verfahren zur Segmentierung von Blutgefäßen, umfassend:

Erfassen (200) einer Vielzahl von Bildern (115) mit einer Vielzahl von Voxeln, die axiale Schnitte durch einen Bereich von Interesse darstellen;
Definieren (210) einer Faszienschicht zwischen einem muskulären Bereich und einem subkutanen Bereich durch Definieren einer Grenze zwischen Bildern hoher Intensität und Bildern niedriger Intensität;
Bestimmen einer ersten Markierung und einer zweiten Markierung eines Blutgefäßes, worin sich die erste Markierung auf einer Seite der Faszienschicht und die zweite Markierung auf der anderen Seite der Faszienschicht befindet;
Berechnen (215) eines subkutanen Pfades zwischen dem ersten Merkmal des Blutgefäßes und der Faszienschicht durch ein automatisches Tracking-Verfahren, umfassend die Analyse der Gefäßrichtung entlang des Blutgefäßes; und
Berechnen (220) eines intramuskulären Pfades zwischen der Faszienschicht und der zweiten Markierung durch Analyse der Voxel und automatisches Bestimmen eines kostengünstigsten Pfades, der durch die Rohrcontinuität der Voxel gegeben ist.

2. Verfahren nach Anspruch 1, ferner umfassend das Reduzieren von Graustufen in der erfassten Vielzahl von Bildern zu binären Bildern.

3. Verfahren nach einem der obigen Ansprüche, ferner umfassend das Entfernen (510, 560) von Artefakten aus der Vielzahl von Bildern.

4. Verfahren nach Anspruch 3, bei welchem die Artefakte im Bild Pixel umfassen, die sich auf die Haut beziehen, freie Stellen in einem ansonsten kontinuierlichen Merkmal.

5. Verfahren nach einem der obigen Ansprüche, bei welchem die Analyse der Gefäßrichtung durch Analyse von lokalen Gradientenvektoren geschätzt wird.

6. Verfahren nach einem der obigen Ansprüche, bei welchem das Zentrum des Blutgefäßes aus Änderungen der Intensität der Voxel in der Vielzahl der erfassten Bilder bestimmt wird.

7. Verfahren nach einem der obigen Ansprüche, bei welchem die Rohrkontinuität der Voxel nach dem Frangi-Verfahren bestimmt wird.

8. Vorrichtung zur Segmentierung eines Blutgefäßes, umfassend:

eine Datenbank (110) zum Speichern einer Vielzahl von Bildern (115) von axialen Schnitten eines Bereichs von Interesse;
einen Prozessor (130), der konfiguriert ist, um die Vielzahl von Bildern mit einer Software zu analysieren, die das Verfahren nach einem der Ansprüche 1 bis 7 ausführt; und
eine Anzeigevorrichtung (140) zum Ausgeben von Ergebnissen aus der Software.

9. Computerprogrammprodukt, das auf einem nicht materiellen, computerlesbaren Medium gespeichert ist und eine Vielzahl von Anweisungen umfasst, die, wenn sie von einem Prozessor (130) ausgeführt werden, den Prozessor (130) veranlassen, das Verfahren nach einem der Ansprüche 1 bis 7 durchzuführen.

## Revendications

1. Procédé de segmentation de vaisseaux sanguins comprenant :

acquérir (200) une pluralité d'images (115) avec une pluralité de voxels représentant des tranches axiales à travers une région d'intérêt ;
définir (210) une couche de fascia entre une région musculaire et une région sous-cutanée en définissant une limite entre des images à haute intensité et des images à faible intensité ;
déterminer un premier repère et un deuxième repère d'un vaisseau sanguin, dans lequel le premier repère se trouve d'un côté de la couche de fascia et le deuxième repère se trouve de l'autre côté de la couche de fascia ;
calculer (215) un chemin sous-cutané entre le premier repère du vaisseau sanguin et la couche de fascia par une procédure de suivi automatique comprenant une analyse de direction de vaisseau le long du vaisseau sanguin ; et
calculer (220) un chemin intramusculaire entre la couche de fascia et le deuxième repère en analysant les voxels et en déterminant automatiquement un itinéraire le moins coûteux donné par la tube-continuité des voxels.

2. Procédé selon la revendication 1, comprenant en outre la réduction des niveaux de gris dans la pluralité acquise d'images en images binaires.

3. Procédé selon l'une quelconque des revendications précédentes, comprenant en outre le retrait (510, 560) d'artefacts de la pluralité d'images.

4. Procédé selon la revendication 3, dans lequel les artefacts dans l'image comprennent des pixels relatifs à la peau, des vides dans une caractéristique autrement continue.

5. Procédé selon l'une quelconque des revendications précédentes, dans lequel l'analyse de la direction du vaisseau est estimée par analyse de vecteurs de gradient locaux.

6. Procédé selon l'une quelconque des revendications précédentes, dans lequel le centre du vaisseau sanguin est déterminé à partir des changements d'intensité des voxels dans la pluralité d'images acquises.

7. Procédé selon l'une quelconque des revendications précédentes, dans laquelle la tube-continuité est déterminée selon la méthode de Frangi.

8. Appareil pour la segmentation d'un vaisseau sanguin comprenant :

une base de données (110) pour stocker une pluralité d'images (115) de coupes axiales d'une région d'intérêt ;
un processeur (130) configuré pour analyser la pluralité d'images avec un logiciel exécutant le procédé selon l'une quelconque des revendications 1 à 87 ; et
un dispositif d'affichage (140) pour sortir les résultats du logiciel.

9. Produit programme d'ordinateur stocké sur un support lisible par ordinateur non tangible et comprenant une pluralité d'instructions, qui, lorsqu'exécutées par un processeur (130), amène le processeur (130) à exécuter le procédé de l'une quelconque des revendications 1 à 7.

# Fig 1

# Fig 3

rectus muscle

anterior lamella of
the rectus sheath
(anterior fascia)

posterior lamella of
the rectus sheath

location where the
DIEA enters the
posterior rectus sheath

end of
perforators

30a

30b

skin

32

Fig 2

200 Take images

205 Define volume of Interest

210 Segment anterior fascia of muscle

215 Obtain subcutaneous path

220 Obtain intramuscular part

225 Generate report

Fig. 4

Fig. 6

Fig. 5

Fig. 7

Fig. 8

Fig. 9

Fig. 10

Fig. 11

Fig. 12

Fig. 13

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014162263 A
- US 8768436 B

**Non-patent literature cited in the description**

- **LANGE et al.** Automating Perforator Flap MRA and CTA Reporting. *J. Digit. Imaging,* 2017, vol. 30, 350-357
- **SIEGEL, R. ; MILLER, K. ; JEMAL, A.** Global cancer statistics. *A Cancer Journal for Clinicians,* 2015, vol. 65 (5), 29
- **HEWITT, M. ; HERDMAN, R. ; HOLLAND, J.** Meeting psychosocial needs of women with breast cancer. The National Academies Press, 2004
- **LICHTER, A. ; LIPPMAN, M. ; DANFORTH, D. ; ANGELO, T. ; STEINBERG, S. ; DEMOSS, E. ; MAC-DONALD, H. ; REICHERT, C. ; MERINO, M. ; SWAIN, S.** Mastectomy versus breast-conserving therapy in the treatment of stage I and II carcinoma of the breast: a randomized trial at the National Cancer Institute. *Journal of Clinical Oncology,* 1992, vol. 10, 976-983
- **MCGUIRE, K. ; SANTILLAN, A. ; KAUR, P. ; MEADE, T. ; PARBHOO, J. ; MATHIAS, M. ; SHAMEHDI, C. ; DAVIS, M. ; RAMOS, D. ; COX, C.** Are Mastectomies on the Rise? A 13-Year Trend Analysis of the Selection of Mastectomy Versus Breast Conservation Therapy in 5865 Patients. *Annals of Surgical Oncology,* 2009, vol. 16, 2682-2690
- **DRAGUN, A. ; HUANG, B. ; TUCKER, T. ; SPANOS, W.** Increasing mastectomy rates among all age groups for early stage breast cancer: A 10-year study of surgical choice. *Breast Journal,* 2012, vol. 18, 318-325
- **MAHMOOD, U. ; HANLON, A. ; KOSHY, M. ; BURAS, R. ; CHUMSRI, S. ; TKACZUK, K. ; CHESTON, S. ; REGINE, W. ; FEIGENBERG, S.** Increasing national mastectomy rates for the treatment of early stage breast cancer. *Annals of surgical oncology,* 2013, vol. 20, 1436-43
- **WEXELMAN, B. ; SCHWARTZ, J. ; LEE, D. ; ESTABROOK, A. ; MA, A.** Socioeconomic and Geographic Di_erences in Immediate Reconstruction after Mastectomy in the United States. *The Breast Journal,* 2014, vol. 20, 339-346

- **CINA, A. ; SALGARELLO, M. ; BARONE-ADESI, L. ; RINALDI, P. ; BONOMO, L.** Planning breast reconstruction with deep inferior epigastric artery perforating vessels: multidetector CT angiography versus Color Doppler US. *Radiology,* 2010, vol. 255, 979-987
- **PHILLIPS, T. ; STELLA, D. ; ROZEN, W. ; ASHTON, M. ; TAYLOR, G.** Abdominal wall CT angiography: a detailed account of a newly established preoperative imaging technique. *Radiology,* 2008, vol. 249, 32-44
- **LESAGE, D. ; ANGELINI, E. ; BLOCH, I. ; FUNKA-LEA, G.** A review of 3D vessel lumen segmentation techniques: Models, features and extraction schemes. *Medical Image Analysis,* 2009, vol. 13, 819-845
- **OTSU, N.** A threshold selection method from gray-level histograms. *IEEE Transactions on Systems, Man, and Cybernetics,* 1979, vol. 9, 62-66
- **AGAM, G. ; ARMATO, S. ; WU, C.** Vessel tree reconstruction in thoracic CT scans with application to nodule detection. *IEEE Transactions on Medical Imaging,* 2005, vol. 24, 486-499
- **KALMAN, R.** A New Approach to Linear Filtering and Prediction Problems. *Journal of Basic Engineering,* 1960, vol. 82, 35-45
- **OLIVEIRA, H. ; CARDOSO, J. ; MAGALHES, A. ; CARDOSO, M.** A 3D low-cost solution for the aesthetic evaluation of breast cancer conservative treatment. *Computer Methods in Biomechanics and Biomedical Engineering: Imaging & Visualization,* 2014, vol. 2, 90-106
- **HART, P. ; NILSSON, N. ; RAPHAEL, B.** A formal basis for the heuristic determination of minimum cost paths. *IEEE Transactions on Systems, Science, and Cybernetics,* 1968, vol. 4, 100-107
- **FRANGI, A. ; NIESSEN, W. ; VINCKEN, K. ; VIERGEVER, M.** Multiscale vessel enhancement Filtering. Medical Image Computing and Computer-Assisted Intervention. *Lecture Notes in Computer Science,* 1998, vol. 1496, 130-137

- **KOVESI, P.** Phase preserving denoising of images. *DICTA 99: Fifth International/National Biennial Conference on Digital Image Computing, Techniques and Applications,* 1999, 212-217

- **BULLITT, E. ; GERIG, G. ; PIZER, S. ; LIN, W. ; AYLWARD, S.** Measuring tortuosity of the intracerebral vasculature from MRA images. *IEEE Trans. Med. Imaging.,* 2003, vol. 22, 1163-1171